# EUROPÄISCHE PATENTANMELDUNG

(11) **EP 1 972 267 A1**
(43) Veröffentlichungstag der Anmeldung: **24.09.2008**
(21) Anmeldenummer: 07005638.7
(22) Anmeldetag: 20.03.2007
(51) Int. Cl.: A61B 5/00

(54) **System zur in-vivo Messung einer Analytkonzentration**

(71) Anmelder: Roche Diagnostics GmbH, 68305 Mannheim (DE); F.HOFFMANN-LA ROCHE AG, 4070 Basel (CH)
(72) Erfinder: Roesicke, Bernd, 68305 Mannheim (DE); Obermeier, Karin, 68782 Brühl (DE); Lindegger, Stefan, 4932 Lotzwil (CH); Menke, Andreas, 68305 Mannheim (DE); Scherer, Jörg, 4528 Zuchwil (CH); Schwind, Karin, 67105 Schifferstadt (DE); Gaa, Otto, 67551 Worms (DE); Bainczyk, Gregor, 68199 Mannheim (DE); Marquant, Michael, 68309 Mannheim (DE); Niederhäuser, Sandro, CH-4933 Rütschelen (CH); Schoemaker, Michael, 68163 Mannheim (DE)
(74) Vertreter: Twelmeier Mommer & Partner

(57) **Zusammenfassung**

Die Erfindung betrifft ein System zur in-vivo Messung einer Analytkonzentration in einem menschlichen oder tierischen Körper, mit mindestens einem implantierbaren Sensor (3) zum Erzeugen von mit der zu messenden Analytkonzentration korrelierenden Messsignalen, einer an den Sensor (3) anschließbare Basisstation (2), die eine elektronische Auswerteeinheit (47) zum Auswerten von Messsignalen eines angeschlossenen Sensors (3) und einen Sender (31) zum drahtlosen Übertragen von Auswertungsergebnissen enthält, und einem Anzeigegerät (4), das einen Empfänger zum Empfangen der von der Basisstation (2) gesendeten Auswertungsergebnisse und eine Anzeigeeinrichtung (29) zum Anzeigen von Analytkonzentrationswerten aufweist. Erfindungsgemäß ist vorgesehen, dass der Sensor (3) Teil einer austauschbaren Sensorträgereinheit (10) ist, die ein Gehäuse (12) mit einer Schnittstelle zum Anschließen des Sensors (3) an die Basisstation (2) aufweist, wobei die Basisstation (2) ein an die Sensorträgereinheit (10) angepasstes Gehäuse aufweist, das eine Schnittstelle aufweist, die zu der Schnittstelle der Sensorträgereinheit (10) passt, so dass der Sensor (3) durch Ansetzten des Gehäuses der Basisstation (2) an das Gehäuse (12) der Sensorträgereinheit (10) an die Auswerteeinheit (47) der Basisstation (2) angeschlossen wird.

## Beschreibung

Die Erfindung geht aus von einem System zur in-vivo Messung einer Analytkonzentration in einem menschlichen oder tierischen Körper, wie es beispielsweise aus der US 2004/0133164 A1 bekannt ist.

Derartige Systeme zur in-vivo Messung von Analytkonzentrationen umfassen in der Regel austauschbare Sensoren als Austausch- oder Verbrauchskomponenten und eine langlebige Basisstation, an welche die austauschbaren Sensoren angeschlossen werden.

Aufgabe der Erfindung ist es, einen Weg aufzuzeigen, wie bei einem System der eingangs genannten Art die Zuverlässigkeit erhöht und die Handhabung für einen Benutzer erleichtert werden kann.

Diese Aufgabe wird erfindungsgemäß durch ein System mit den Merkmalen des Anspruchs 1 gelöst.

Bei Systemen zur in-vivo Messung von Analytkonzentrationen, beispielsweise Glucose, müssen Sensoren in der Regel im Abstand von wenigen Tagen ausgetauscht werden. Der Austausch eines Sensors und das korrekte Anschließen eines neuen Sensors an die Basisstation gestaltet sich für viele Benutzer, insbesondere für Patienten, deren manuelle Geschicklichkeit durch Alter oder Krankheit eingeschränkt ist, mühsam. Indem der Sensor Teil einer austauschbaren Sensorträgereinheit ist, die ein Gehäuse mit einer Schnittstelle zum Anschließen des Sensors an die Basisstation aufweist, kann der Austausch von Sensoren wesentlich erleichtert werden, so dass ein erfindungsgemäßes System insbesondere auch von medizinischen Laien benutzt werden kann. Der Sensor wird über eine Datenleitung in der Regel elektrisch and an die Basisstation angeschlossen, beispielsweise bei Einsatz von elektrochemischen Sensoren. Bei Verwendung von optischen Sensoren, wie sie beispielsweise aus der US 6,584,335, bekannt sind, kann der Sensor jedoch auch über eine optische Datenleitung an die Basisstation angeschlossen werden.

Bei einem erfindungsgemäßen System weist die Basisstation ein an die Sensorträgereinheit angepasstes Gehäuse auf, das eine Schnittstelle aufweist, die zu der Schnittstelle der Sensorträgereinheit passt, so dass der Sensor durch Ansetzen des Gehäuses der Basisstation an das Gehäuse der Sensorträgereinheit elektrisch an die Basisstation angeschlossen wird. Eine reversible, d. h. wieder lösbare, Ankopplung der Sensorträgereinheit an die Basisstation lässt sich beispielsweise durch eine kraft- oder formschlüssige Verbindung realisieren. Besonders günstig und deshalb bevorzugt ist es dabei, wenn die Sensorträgereinheit mit der Basisstation verrastet, da dies von einem Benutzer wahrgenommen werden kann, so dass ihm dadurch signalisiert wird, dass die Sensorträgereinheit korrekt an die Basisstation angeschlossen ist.

Bei dem Sensor kann es sich beispielsweise um einen amperometrischen Sensor halten, der über einen in der Basisstation enthaltenen Potentiostaten mit Spannung versorgt wird. Für das System können aber auch elektrochemische Sensoren verwendet werden, die keinen Potentiostaten benötigen, beispielsweise coulombmetrische Sensoren, oder beispielsweise auch optische Sensoren.

Um das Gewicht der am Körper getragenen Systemkomponenten möglichst gering zu halten, ist ein möglichst sparsamer Energieverbrauch der Basisstation vorteilhaft, da dann eine kleinere und leichtere Batterie ausreicht, um diese Systemkomponente über eine hinreichend lange Zeit mit Energie zu versorgen. Ein weiterer Aspekt der Erfindung, der auch selbstständige Bedeutung haben kann, betrifft deshalb ein System zur in-vivo Messung einer Analytkonzentration in einem menschlichen oder tierischen Körper, mit mindestens einem implantierbaren Sensor zum Erzeugen von mit der zu messenden Analytkonzentration korrelierenden Messsignalen, einer an den Sensor anschließbare Basisstation, die einen Potentiostaten zur Spannungsversorgung eines derartigen Sensors sowie einen Empfänger und einen Sender zum drahtlosen Übertragen von Daten enthält, wobei die Basisstation derart eingerichtet, dass das Aussenden von Daten durch den Empfang eines drahtlos gesendeten Steuerungssignals veranlasst wird. Um Fehlkommunikationen mit systemfremden Geräten zu vermeiden, kann das Steuerungssignal eine charakteristische Kennung enthalten, mit der sich das abfragende Gerät gegenüber der Basisstation identifiziert. In entsprechender Weise kann auch die Basisstation bei der Kommunikation ein charakteristisches Kennungssignal aussenden, um sich zu identifizieren.

Bei den Daten kann es sich insbesondere um verdichtete Messdaten handeln, die eine in der Basisstation enthaltene Auswerteeinheit aus Rohdaten ermittelt hat, die als Messsignale eines angeschlossenen Sensors gewonnen wurden. Ein das Aussenden der Messdaten veranlassendes Steuersignal kann beispielsweise von einem Anzeigegerät gesendet werden.

Mit implantierbaren Sensoren können in sehr kurzen Zeitabständen von beispielsweise einer Sekunde Messsignale erzeugt werden, so dass bei fortlaufender Messung sehr große Mengen an Rohdaten anfallen können. Ein weiterer Aspekt der Erfindung, der auch selbstständige Bedeutung haben kann, betrifft ein Verfahren zum Verdichten von mit einem implantierten Sensor ermittelten Rohdaten, bei dem aus den für ein Zeitintervall erzeugten Rohdaten Paare von Messsignalwerten gebildet werden, danach für jedes Messsignalwertepaar eine Steigung für eine die die beiden Werte des Wertepaares verbindende Linie ermittelt wird, danach ein Medianwert der ermittelten Steigungen berechnet wird und danach für das Zeitintervall ein verdichteter Datenwert aus dem Medianwert der Steigung und dem verdichteten Datenwert des vorhergehenden Zeitintervalls berechnet wird.

Da für das erste Zeitintervalls zunächst kein verdichteter Datenwert vorhanden ist, kann als verdichteter Datenwert des ersten Zeitintervalls beispielsweise der Median oder das arithmetische Mittel der für das erste Zeitintervall ermittelten Rohdatenwerte verwendet werden. Rohdatenwerte, die beispielsweise wegen einer ungewöhnlich starken Abweichung von den übrigen Rohdatenwerten eines Zeitintervalls, unplausibel sind, können bei der Ermittlung des verdichteten Datenwertes unberücksichtigt bleiben, beispielsweise indem sie nicht zur Bildung von Wertepaaren verwendet werden.

Bevorzugt werden die von einem Sensor gelieferten Messsignale als Rohdaten in der Basisstation in einem ersten Auswertungsschritt zu Messdaten verdichtet, die verdichteten Messdaten an das Anzeigegerät übertragen und in einem weiteren Auswertungsschritt aus den Messdaten mittels der Auswerteeinrichtung des Anzeigegeräts Analytkonzentrationswerte berechnet. Solch eine zweistufige Auswertung mit einem ersten Auswertungsschritt in der Basisstation und einem weiteren Auswertungsschritt in dem Anzeigegerät ermöglicht es, die Vorteile einer kontinuierlichen oder quasi-kontinuierlichen Messung hinsichtlich Aktualität und Genauigkeit zu nutzen und trotzdem die von der Basisstation zu sendenden Datenmengen klein zu halten. Insbesondere genügen zur Verdichtung der Rohdaten, beispielsweise durch Mittelwertbildung oder Anwendung des repeated median Verfahrens, relativ einfache und deshalb kostengünstige Mikroprozessoren in der Basisstation. Zur abschließenden Auswertung der verdichteten Messdaten kann in dem Anzeigegerät ein leistungsstarker und teuerer Mikroprozessor verwendet werden, der dort auch für andere Aufgaben genutzt werden kann, beispielsweise für eine graphische Darstellung der ermittelten Analytkonzentrationswerte und eine Verknüpfung mit weiteren Daten, die durch das Anzeigegerät erzeugt und gespeichert wurden oder aus anderen Quellen stammen. Ein weiterer Aspekt der Erfindung, der auch selbstständige Bedeutung haben kann, betrifft deshalb ein System zur in-vivo Messung einer Analytkonzentration in einem menschlichen oder tierischen Körper, mit mindestens einem implantierbaren Sensor zum Erzeugen von mit der zu messenden Analytkonzentration korrelierenden Messsignalen, einer an den Sensor anschließbare Basisstation, die eine elektronische Auswerteeinheit zum Auswerten von Messsignalen eines angeschlossenen Sensors und einen Sender zum drahtlosen Übertragen von Auswertungsergebnissen enthält, und einem Anzeigegerät, das einen Empfänger zum Empfangen der von der Basisstation gesendeten Auswertungsergebnisse und eine Anzeigeeinrichtung zum Anzeigen von Analytkonzentrationswerten aufweist, wobei die Auswerteeinheit der Basisstation im Betrieb die von einem angeschlossenen Sensor gelieferten Rohdaten statistisch auswertet und aus den Rohdaten verdichtete Messdaten erzeugt, die über den Sender zu dem Anzeigegerät übertragen werden, und das Anzeigegerät eine elektronische Auswerteeinheit enthält, die im Betrieb durch Auswertung der Messdaten einen Analytkonzentrationswert ermittelt.

Weitere Einzelheiten und Vorteile der Erfindung werden anhand von Ausführungsbeispielen unter Bezugnahme auf die beigefügten Zeichnungen erläutert. Gleiche und einander entsprechende Komponenten sind dabei mit übereinstimmenden Bezugszeichen gekennzeichnet. Die im Folgenden beschriebenen Merkmale können einzeln oder in Kombination zum Gegenstand von Ansprüchen gemacht werden. Es zeigen:
- Fig. 1: eine schematische Darstellung eines Ausführungsbeispiels eines erfindungsgemäßen Systems zur in-vivo Messung einer Analytkonzentration in einem menschlichen oder tierischen Körper;
- Fig.2: eine schematische Darstellung des Zusammenwirkens der Systemkomponenten des in Fig. 1 gezeigten Ausführungsbeispiels;
- Fig. 3: ein Ausführungsbeispiel einer Basisstation und einer daran angeschlossenen Sensorträgereinheit eines Systems gemäß Fig. 1;
- Fig. 4: eine Querschnittsdarstellung zu Figur 3; und
- Fig. 5: ein Sensorgehäuse des in den Figuren 3 und 4 dargestellten Ausführungsbeispiels.

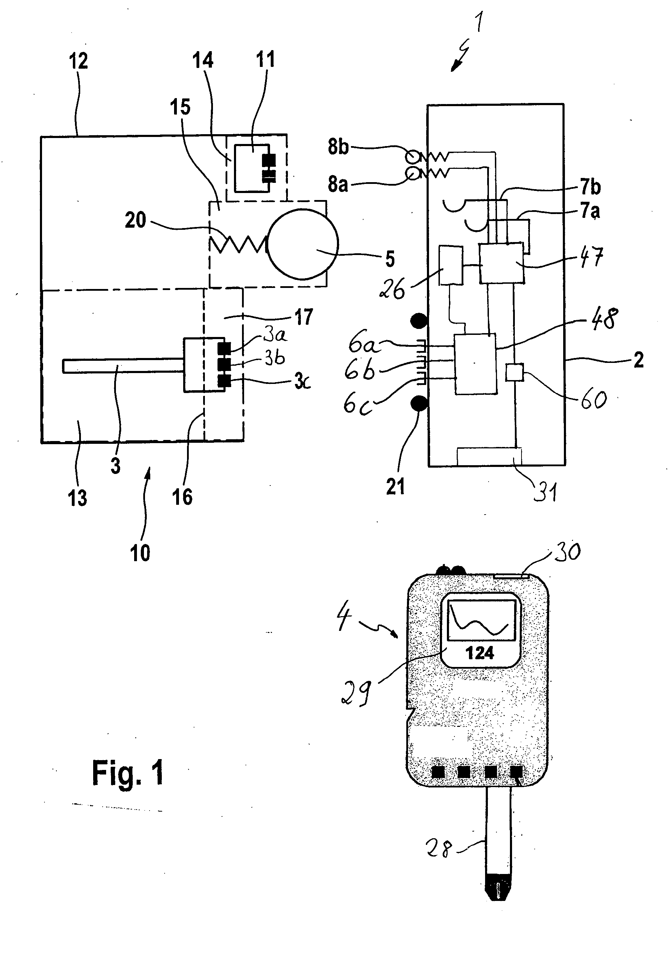

Fig. 1 zeigt in einer schematischen Darstellung ein Systems 1 zur in-vivo Messung einer Analytkonzentration in einem menschlichen oder tierischen Körper.

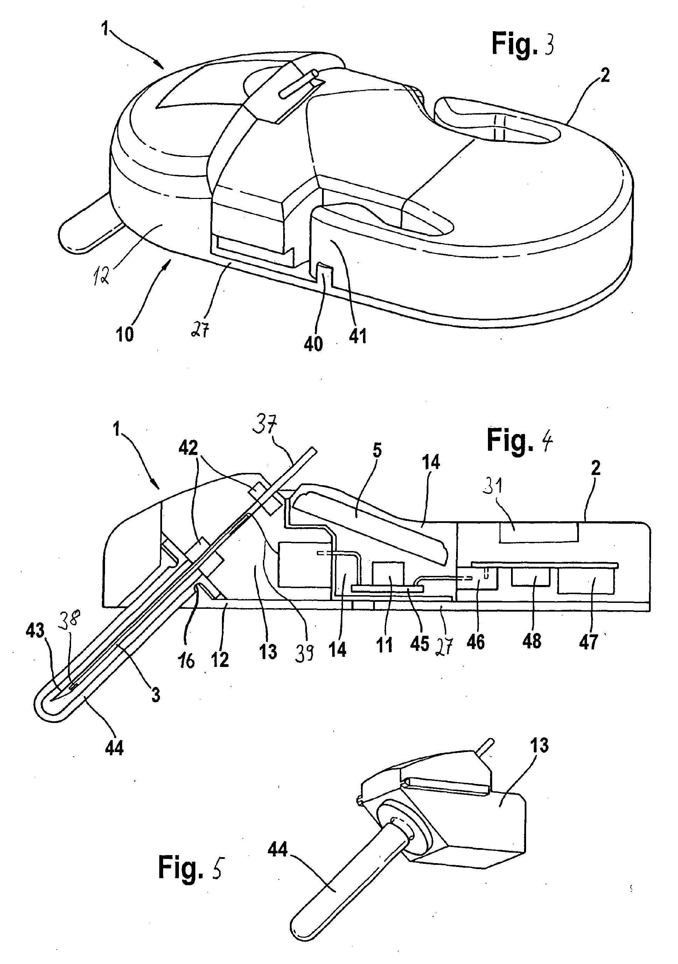

Zu dem System 1 gehört als Verbrauchs- oder Austauschkomponente eine Sensorträgereinheit 10 mit mindestens einem implantierbaren Sensor 3 zum Erzeugen von mit der zu messenden Analytkonzentration korrelierenden Messsignalen. Die Sensorträgereinheit 10 hat ein in Figur 3 dargestelltes Gehäuse 12 mit einem Boden 27, der sich bei bestimmungsgemäßer Befestigung der Sensorträgereinheit 10 am Körper eines Patienten entlang dessen Hautoberfläche erstreckt. Die Sensorträgereinheit 10 enthält eine Batterie 5 und einen Datenträger 11 mit Kalibrierungsdaten des Sensors 3. Die Batterie 5 und der Datenträger 11 sind für einen Benutzer unzugänglich in dem Gehäuse 12 der Sensorträgereinheit 10 angeordnet, um fehlerhafte Resultate oder Schäden durch unsachgemäße Handhabung möglichst auszuschließen.

Die Sensorträgereinheit 10 kann kraft- oder formschlüssig, insbesondere durch einen Rastmechanismus an eine Basisstation 2 angeschlossen werden. Die Basisstation 2 ist eine mehrfach verwendbare Komponente des Systems 1. Eine verbrauchte Sensorträgereinheit 10 kann von der Basiseinheit 2 abgekoppelt und durch eine neue Sensorträgereinheit 10 ersetzt werden. Die Basisstation 2 enthält einen Potentiostaten 48 zur Spannungsversorgung eines Sensors 3 einer angeschlossenen Sensorträgereinheit 10, eine elektronische Auswerteeinheit 47 zum Auswerten von Messsignalen eines angeschlossenen Sensors 3 und eine Kommunikationseinheit 31 mit einem Sender zum drahtlosen Übertragen von Auswertungsergebnissen. Die Basisstation 2 enthält ferner einen elektronischen Zwischenspeicher 60 zur Speicherung von Messwerten oder daraus gewonnenen Daten. Bei dem dargestellten Auswertungsbeispiel enthält die Basisstation 2 einen Speicher zur Zwischenspeicherung von Rohdaten, beispielsweise einen RAM Speicher, und einen Speicher zur Speicherung von verdichteten Messdaten, beispielsweise einen EEPROM oder Flash-Speicher, bis zu deren Aussenden und/oder zur Speicherung von Daten, die die Basisstation 2 von dem Anzeigegerät 4 übergeben wurden.

Die Sensorträgereinheit 10 hat ein Gehäuse 12 mit einer Schnittstelle zum elektrischen Anschließen des Sensors 3 an die Basisstation 2. In entsprechender Weise hat die Basisstation 2 ein an die Sensorträgereinheit 10 angepasstes Gehäuse, das eine Schnittstelle aufweist, die zu der Schnittstelle der Sensorträgereinheit 10 passt, so dass der Sensor 3 elektrisch an den Potentiostaten 48 angeschlossen werden kann, indem das Gehäuse der Basisstation 2 an das Gehäuse 12 der Sensorträgereinheit 10 angesetzt wird, so dass Sensorkontakte 3a, 3b und 3c mit Anschlüssen 6a, 6b und 6c der Basisstation verbunden werden. Dabei werden auch die Batterie 5 und der Datenträger 11 an Kontakte 7a, 7b bzw. 8a, 8b der Schnittstelle der Basisstation 2 angeschlossen. Die zueinander passenden Schnittstellen der Sensorträgereinheit 10 und der Basisstation 2 können eine Steckverbindung bilden, wobei der männliche Teil der Steckverbindung an der Basisstation 2 und der weibliche Teil an der Sensorträgereinheit 10 angeordnet sein kann oder umgekehrt.

Durch das Anschließen der Sensorträgereinheit 10 an die Basisstation 2 wird eine Inbetriebnahme bewirkt, insbesondere der Messprozess des Sensors 3 gestartet. Mit dem Anschließen der Sensorträgereinheit 10 an die Basisstation 2 kann hierfür bei einem die Auswerteeinheit 47 bildenden Prozessor ein Reset- und Initialisierungsbefehl bewirket werden. Diese Maßnahme hat den Vorteil, dass kein Schalter zum Einschalten des Systems benötigt wird und die Inbetriebnahme des Systems durch eine definierte Handlung des Benutzers, nämlich dem Anschließen einer Sensorträgereinheit 10 an die Basisstation 2, bewirkt wird. Sobald durch Anschließen der Batterie an dem Potentiostaten 48 eine Spannung anliegt, werden Ladensträger automatisch an der Arbeitselektrode abtransportiert, so dass die Gefahr von Potentialverschiebungen minimiert ist.

Als weitere langlebige Komponente gehört zu dem System ein Anzeigegerät 4, das einen Empfänger 30 zum Empfangen der von der Basisstation 2 gesendeten Daten und eine Anzeigeeinrichtung 29, beispielsweise eine Flüssigkristallanzeige, zum Anzeigen von Analytkonzentrationswerten aufweist. Bevorzugt enthält der Empfänger 30 des Anzeigegeräts 4 auch einen Sender und der Sender 31 der Basisstation auch einen Empfänger, so dass eine bidirektionale Kommunikation zwischen Basisstation 2 und Anzeigegerät 4 möglich ist. Sendungen der Basisstation 2 und des Anzeigegeräts 4 können durch eine charakteristische Kennung, beispielsweise eine Bitfolge, gekennzeichnet werden. Um Fehlkommunikationen mit Geräten anderer Patienten auszuschließen, können Signale, die nicht die erwartete Kennung tragen, von der Basisstation 2 und dem Anzeigegerät 4 ignoriert werden.

Durch die beschriebene Aufteilung von Funktionen und Komponenten auf die Systembestandteile Sensorträgereinheit 10, Basisstation 2 und Anzeigegerät 4 kann hinsichtlich Gewicht der am Körper zu tragenden Systembestandteile, Kosten und Benutzerkomfort ein optimales Ergebnis erzielt werden. Verbrauchskomponenten des Systems, beispielsweise Sensor 3 und Batterie 5, sind Teil der Sensorträgereinheit 10, die periodisch, beispielsweise alle 5 Tage ausgetauscht werden muss, so dass alle Verbrauchskomponenten möglichst einfach gewechselt werden können. Um mit einem Sensor 3 der Sensorträgereinheit 10 Messdaten zu erzeugen, werden neben den Verbrauchskomponenten auch langlebige Systemkomponenten, wie beispielsweise der Potentiostat 48 und eine Auswerteeinheit benötigt. Die Aufteilung dieser langlebigen Systemkomponenten auf die ebenfalls am Körper getragene Basisstation 2 und das Anzeigegerät 4 wurde im Hinblick auf einen möglichst großen Benutzerkomfort vorgenommen, um die Basisstation 2 möglichst klein und leicht verwirklichen zu können. Die Basisstation 2 enthält deshalb einen Potentiostaten 48 zur Versorgung eines angeschlossenen Sensors 3, einen Sender zur Datenübertragung an das Anzeigegerät 4 und eine Auswerteeinheit 48 für eine Vorauswertung der von einem angeschlossenen Sensor gelieferten Messdaten. Die Basisstation 2 benötigt keine eigene Anzeigeeinrichtung, da diese Funktion von dem Anzeigegerät 4 übernommen wird. Eine Vorauswertung in der Basisstation 2 in Kombination mit einer Endauswertung in dem Anzeigegerät 4 ermöglicht es, den Umfang der zu senden Datenmengen klein zu halten. Die Basisstation 2 benötigt deshalb nur eine kleinen Speicher, eine einfache Auswerteeinheit und wenig Energie, so dass ein Patient möglichst wenig Masse am Körper tragen muss.

Das Anzeigegerät 4 kann mit einem großen Speicher und einer komplexen Auswerteelektronik, insbesondere einem leistungsstarken Mikroprozessor, ausgerüstet werden und deshalb auch mathematisch aufwändige Auswertungen von Daten über lange Zeiträume vornehmen. Insbesondere kann das Anzeigegerät 4 bei einem neu implantierten Sensor durch Vergleich mit Messdaten früherer Sensoren oder festgelegten Pegel- bzw. Gradientenwerten und Formfaktoren oder Mustern prüfen, ob der neue Sensor korrekt funktioniert. Danben ermöglicht das Anzeigegerät 4 seinem Benutzer auch eine komfortable Eingabe von Patientendaten, beispielsweise individuelle Schwellenwerte, bei deren Über- bzw. Unterschreiten von dem Anzeigegerät 4 ein Warnsignal erzeugt werden kann. Das Anzeigegerät 4 kann zusätzlich zu einer Auswertungs-, Anzeige- und Warnfunktion auch die Funktion eines Daten- und Kommunikationszentrums in Bezug auf weitere Komponenten des Systems, beispielsweise ein Injektionsgerät übernehmen.

Die Basisstation 2 wird bestimmungsgemäß von einem Patienten während der in-vivo Messungen zusammen mit der Sensorträgereinheit 10 am Körper getragen. Der elektrochemische Sensor 3 ragt dabei in den Körper des Patienten hinein und wird von dem in der Basiseinheit enthaltenen Potentiostaten 48 versorgt. Beim Messen fließt zwischen der Arbeitselektrode und der Gegenelektrode des Sensors 3 ein Strom, dessen Stärke mit der zu messenden Analytkonzentration korreliert, im Idealfall zu ihr proportional ist. Der Potentiostat 48 variiert dabei das an der Gegenelektrode anliegende elektrische Potential derart, dass das Potential der Referenzelektrode des Sensors 3 konstant bleibt.

Mit dem dargestellten System 1 kann eine Analytkonzentration im Körper eines Patienten kontinuierlich oder quasikontinuierlich überwacht werden. Dies bedeutet, dass mit dem Sensor 3 in kurzen Zeitabständen von weniger als 5 Minuten, insbesondere weniger als 1 Minute oder sogar weniger als 10 Sekunden, Messungen vorgenommen werden können. Eine Messung kann dabei durch digitalisieren eines Rohsignals, beispielsweise einer elektrischen Stromstärke, erfolgen, um ein Messsignal zu erzeugen. Parallel zu diesen Messungen können weitere Daten gesammelt werden, beispielsweise Temperatur und/oder Elektrodenspannungen, die zur Plausibilitätsprüfung und/oder Fehlkompensation herangezogen werden können.

Bei dem beschriebenen System 1 werden in Zeitabständen von etwa einer Sekunde, beispielsweise zwischen 0,5 und 2 Sekunden, Messungen vorgenommen, so dass sehr große Mengen an Rohdaten anfallen. Bei den von dem Sensor 3 in derartigen Zeitabständen gelieferten Messsignalen kann es sich um Werte eines kontinuierlichen Signals handeln, das bereits verstärkt und/oder gefiltert wurde, beispielsweise mit einem Tiefpass, um elektrische Störungen, wie sie durch die Frequenz des öffentlichen Stromnetzes (50 Hz bzw. 60 Hz), Rauschen oder Funkverkehr entstehen können, herauszufiltern. Die Grenzfrequenz eines solchen Tiefpasses liegt vorzugsweise zwischen 3 Hz und 50 Hz, insbesondere zwischen 5 Hz und 20 Hz. Unter der Grenzfrequenz wird dabei gemäß dem allgemeinen Sprachgebrauch die Frequenz verstanden, bei welcher der Tiefpass eine Dämpfung von 3 dB bewirkt.

Niederfrequente Störungen der Messsignale, die beispielsweise aus Störungen der Elektrochemie an Grenzflächen zwischen dem Sensor und umgebendem Körpergewebe entstehen können, können durch Filteralgorithmen entfernt werden, beispielsweise das in der Anmeldung beschrieben Repeated Median Verfahren.

Die im Betrieb von einem Sensor 3 gelieferten Messsignale werden von der in der Basisstation 2 enthaltenen Auswerteeinheit 47 einer Vorauswertung unterzogen. Dabei werden die Messsignale als Rohdaten statistisch ausgewertet und so aus den Rohdaten verdichtete Messdaten erzeugt. Die verdichteten Messdaten werden von der Basiseinheit 2 für konstante, aufeinander folgende Zeitintervalle von beispielsweise einer Minute erzeugt, so dass aus deren Abfolge und der Größe der Zeitintervalle für die Messdaten eine eindeutige Zeitzuordnung gegeben ist. Die verdichteten Messdaten werden dann drahtlos zu dem Anzeigegerät 4 übertragen und dort zur Ermittelung von Analytkonzentrationswerten mittels einer Auswerteeinheit, beispielsweise einem Mikroprozessor, weiter ausgewertet.

Bei dem dargestellten Ausführungsbeispiel enthält die Basisstation 2 einen Speicher, in dem die verdichteten Messdaten abgelegt werden können, so dass sie nicht unmittelbar nach Erzeugen gesendet werden müssen. Die gespeicherten Datensätze können mit einem Prüfcode versehen werden, der es ermöglicht, die verdichteten Messdaten in späteren Auswertungsschritten auf Datenkorruption zu prüfen und fehlerhafte Messdaten zu erkennen. Zusammen mit den Messdaten können in dem Speicher auch Zustandsinformationen der Basiseinheit 2, beispielsweise Ladezustand der Batterie, Ergebnisse interner Funktionsprüfungen und ähnliches, gespeichert werden. Diese Zustandsinformationen können als Statuscode, beispielsweise als Byte, abgespeichert und bei der Auswertung berücksichtigt werden.

Eine besonders effiziente Speicherung der verdichteten Messdaten lässt sich dadurch erreichen, dass als Messdatenwert nur die Abweichung von einem vorhergehenden Messdatenwert gespeichert wird. Auf diese Weise genügt es, den ersten Messdatenwert in dem Speicher vollständig zu speichern. Alle folgenden Werte können durch ihrer Differenz zu dem vorhergehenden Messdatenwert eindeutig gekennzeichnet werden, so dass die Speicherung dieser Differenz genügt.

Die zu verdichtenden Messsignale werden als Rohdaten in der Basisstation nur vorübergehend gespeichert, bis die verdichteten Messdaten erzeugt wurden. Die Basisstation 2 weist deshalb einen Rohdatenspeicher auf, dessen Inhalt überschrieben wird, sobald für die Messsignale eines Zeitintervalls ein verdichteter Messdatenwert ermittelt wurde und die Messsignale nicht mehr benötigt werden. Für manche Anwendungen kann es vorteilhaft sein, eine spätere Zugriffsmöglichkeit auf die Rohdaten zu bieten. Dies kann beispielsweise dadurch erreicht werden, dass Rohdaten vor dem Überschreiben gesendet werden. Diese Sendung kann unidirektional erfolgen, d.h. ohne dass ihr Empfang von einem Empfangsgerät, das die Rohdaten speichert durch ein Empfangssignal bestätigt wird. Die Basisstation 2 kann für undirektionale Sendungen einen weiteren Sender aufweisen.

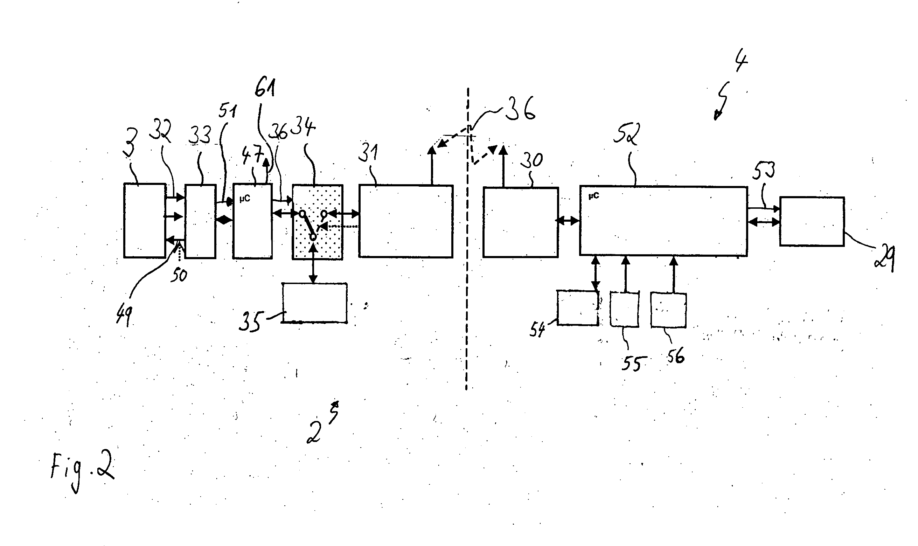

In Figur 2 ist schematisch das Zusammenwirken der verschiedenen Systemkomponenten dargestellt. Links der gestrichelten Linie sind der Sensor 3 und Komponenten einer daran angeschlossene Basisstation 2, rechts der gestrichelten Linie Komponenten des Anzeigegeräts 4 dargestellt.

Im Betrieb wird der Sensor 3 von der Basisstation 2 mit Spannung 49 versorgt, deren Höhe von Kalibrierungsdaten 50 abhängen kann, die auf dem in Figur 1 dargestellten Datenträger 11 gespeichert sind. Der Sensor 3 liefert Messsignale 32, die von einem Alalog-Digital Konverter 33 digitalisiert werden und anschließend als digitale Rohdaten 51 von der Auswerteeinheit 47, bevorzugt einem Mikroprozessor, statistisch ausgewertet werden. Dabei werden aus den Rohdaten 51 verdichtete Messdaten 36 erzeugt. Die verdichteten Messdaten 36 werden in einem Speicher 35 abgelegt, auf den die Auswerteeinheit 47 ebenso wie die einen Sender und einen Empfänger enthaltende Kommunikationseinheit 31 zu greifen kann. Zur Vermeidung von Zugriffskonflikten sind die Auswerteeinheit 47 und die Kommunikationseinheit 31 an den Speicher 35 über einen Umschalter 34 angeschlossen, der je nach Schaltzustand entweder der Auswerteeinheit 47 oder der Kommunikationseinheit 31 Zugriff auf den Speicher 35 ermöglicht.

Die Kommunikationseinheit 31 der Basisstation 2 liest die verdichteten Messdaten 36 aus dem Speicher 35 und sendet diese an eine Kommunikationseinheit 30 des Anzeigegeräts 4. Bei dem dargestellten Ausführungsbeispiel enthält die Kommunikationseinheit 30 einen Sender und einen Empfänger, so dass ein bidirektionaler Datenaustausch möglich ist. Möglich ist es aber auch, dass die verdichteten Messdaten 36 von der Kommunikationseinheit 31 der Basisstation 2 unidirektional gesendet werden. Insbesondere ist es möglich, die Auswerteeinheit 47 mit einem Sender 61 zum undirektionaleh Aussenden von Rohdaten auszustatten.

Die verdichteten Messdaten 36 werden in dem Anzeigegerät 4 von der darin enthaltenen Auswerteeinheit 52, beispielsweise einem Mikroprozessor, ausgewertet, um Analytkonzentrationswerte 53 zu ermitteln, die von der Anzeigeeinrichtung 29 angezeigt und ebenso wie die verdichteten Messdaten 36 in einem Speicher 54 gespeichert werden können. Beim Auswerten der verdichteten Messdaten 36 führt die Auswerteeinheit 52 des Anzeigegeräts 4 auch eine Kalibrierung unter Berücksichtigung von Messergebnissen eines Messgeräts 56 durch, das in das Anzeigegerät 4 integriert ist und die Analytkonzentration einer Körperflüssigkeitsprobe bestimmt, die beispielsweise aus einer kleinen Stichwunde gewonnen wurde.

Bei der Auswertung der verdichteten Messdaten 36 kann die Auswerteeinheit 52 des Anzeigegeräts 4 mittels einer Echtzeituhr 55 zu den verdichteten Messdaten und den daraus ermittelten Analytkonzentrationswerten jeweils eine Uhrzeit und ein Datum, da die verdichteten Messdaten für Zeitintervalle konstanter Dauer gelten.

Bei der statistischen Auswertung der Messsignale in der Basisstation 2 kann es sich im einfachsten Fall um eine Mittelwertbildung handeln. Die statistische Auswertung kann aber auch Filter- und/oder Korrekturalgorithmen enthalten, um fehlerhafte Werte durch Filterung von der weiteren Auswertung auszuschließen oder zu korrigieren. Geeignet sind beispielsweise Kalman-Filterverfahren.

Als Verfahren zum Filtern und Verdichten der Rohdaten kann ein repeated median Verfahren eingesetzt werden. Bei einem solchen Verfahren wird der Median der Steigungen zwischen Paaren von Messsignalwerten eines Zeitintervalls gebildet. Bei dieser Medianbildung können extreme und deshalb unplausible Rohdatenwerte unberücksichtigt bleiben, beispielsweise Werte, die um mehr als einen vorgegebenen Schwellenwert von dem Durchschnittswert aller übrigen Rohdatenwerte des Zeitintervalls abweichen. Möglich ist es aber auch bei der Medianbildung tatsächlich alle möglichen Wertepaare, die aus den Rohdatenwerten des Zeitintervalls gebildet werden können, bei der Ermittlung des Median der Steigung zu berücksichtigen.

Aus dem Medianwert der Steigung und dem verdichteten Messdatenwert für das vorhergehende Zeitintervall wird dann ein Messdatenwert für das aktuelle Zeitintervall berechnet. Hierfür kann zu dem Messdatenwert des vorhergehenden Zeitintervalls das Produkt aus dem Medianwert der Steigung und der Dauer des Zeitintervalls addiert werden. Für das allererste Zeitintervall kann als verdichteter Messdatenwert beispielsweise der Median der in dem Zeitintervall enthaltenen Rohdatenwerte verwendet werden.

Bei dem beschriebenen repeated median Verfahren ist es günstig, die Zeitintervalle, für die jeweils ein verdichteter Messdatenwert aus dem Median der Steigung und dem vorhergehenden Messdatenwert berechnet wird, überlappend zu wählen. Möglich ist es insbesondere auch, das repeated median Verfahren mehrfach anzuwenden, also beispielsweise in einem ersten Schritt aus Rohdatenwerten verdichtete oder komprimierte Messdaten zu erzeugen und in einem weiteren Schritt die komprimierten Messdatenwerte durch nochmalige Anwendung des repeated median Verfahrens weiter zu komprimieren.

Durch eine zweistufige Auswertung, bei der eine erste Auswertungsstufe in der Basisstation 2 und eine zweite Auswertungsstufe in dem Anzeigegerät 4 vorgenommen wird, können auch sehr große Mengen von Rohdaten verarbeitet werden und so die Vorteile einer kontinuierlichen oder quasi kontinuierlichen Messung hinsichtlich Aktualität und Genauigkeit voll ausgeschöpft werden. Trotzdem müssen von der Basisstation 2 an das Anzeigegerät 4 nur kleine Datenvolumen gesendet werden. Der Energieverbrauch des am Körper getragenen Basisteils 2 ist deshalb gering, so dass eine kleine, leichte und kostengünstige Batterie 5 ausreicht, um den Energiebedarf des Basisteils 2 und der Sensorträgereinheit 10 zu decken. Das zweistufige Auswertungsverfahren trägt deshalb dazu bei, das Gewicht, das der Patient am Körper tragen muss, möglichst weit zu reduzieren und so den Benutzerkomfort zu erhöhen.

Um den Datenverkehr zwischen der Basisstation 2 und dem Anzeigegerät 4 zu minimieren, könnte man in der Basisstation 2 auch eine vollständige Auswertung der Rohdaten vornehmen, so dass an das Anzeigegerät 4 nur noch Analytkonzentrationswerte übertragen werden. Bei der beschriebenen zweistufigen Auswertung wird in der Basisstation 2 jedoch nur eine geringe Prozessorleistung benötigt, so dass ein besonders kostengünstiger Mikroprozessor als Auswerteeinheit 47 eingesetzt werden kann. Ein weiterer Vorteil des beschriebenen zweistufigen Auswerteverfahren besteht darin, dass im Rahmen des zweiten Auswertungsschrittes in dem Anzeigegerät 4 eine Kalibrierung der Messdaten anhand einer Kontrollmessung durchgeführt werden kann. Zu diesem Zweck enthält das Anzeigegerät 4 eine Messeinrichtung zur Bestimmung einer Analytkonzentration einer Körperflüssigkeitsprobe. Diese Messeinrichtung kann beispielsweise gemäß handelsüblichen Blutzuckermessgeräten aufgebaut sein und die Glucosekonzentration einer auf einem Teststreifen 28 aufgebrachten Körperflüssigkeitsprobe fotometrisch oder elektrochemisch bestimmen. Indem die Auswerteeinheit des Anzeigegeräts 4 an die Messeinrichtung angeschlossen ist, können die anhand von Körperflüssigkeitsproben bestimmten Analytkonzentrationswerte bei der Auswertung der Basisstation 2 übermittelten Messdaten zur Kalibrierung verwendet werden.

Neben einer Messeinrichtung zur Bestimmung einer Analytkonzentration einer Körperflüssigkeitsprobe kann das Anzeigegerät 4 auch eine Eingabemöglichkeit zum Eingeben von Patientendaten aufweisen. Patientendaten können beispielsweise Informationen über eingenommene Malzeiten oder individuelle Schwellenwerte für die Messdaten, bei deren Über- bzw. Unterschreiten ein Warnsignal erzeugt werden kann. Das Anzeigegerät 4 kann ferner auch zur Kommunikation mit weiteren Systemkomponenten, beispielsweise einem Injektionsgerät, oder einem übergeordneten HOST-System, beispielsweise dem PC eines Artzes, Betreuers oder einer Klink, eingerichtet sein.

Das Anzeigegerät 4 ist mit einem Sender 30 zum Senden von Steuerungssignalen und die Basiseinheit 2 mit einem Empfänger 31 zum Empfang von Steuerungssignalen sowie einem elektronischen Speicher zur Speicherung der verdichteten Messdaten ausgestattet. Das Aussenden der verdichteten Messdaten wird durch ein von dem Anzeigegerät 4 gesendetes Steuersignal veranlasst. Das Anzeigegerät 4 bestätigt den Empfang der Messdaten durch Aussenden eines Bestätigungssignals.

Das Anzeigegerät 4 enthält eine Uhr zum Ermitteln der jeweils aktuellen Zeit. Empfängt das Anzeigegerät 4 verdichtete Messdaten von der Basisstation 2, kann der aktuelle Messdatenwert mit einer aktuellen Zeitmarke, in der die aktuelle Uhrzeit und Datum kodiert sind, markiert und gespeichert werden. Die Zeitmarken vorangehender Messdatenwerte können ausgehend von dieser Zeitmarke berechnet werden. Wird beispielsweise für aufeinander folgende Zeitintervalle von jeweils einer Minute eine verdichteter Messdatenwert erzeugt, kann durch Dekrementieren im Minutenabstand für alle Messdatenwerte die Tageszeit, für welche sie ermittelt wurden, berechnet werden.

Das Anzeigegerät 4 enthält einen Speicher, der groß genug ist, um Messdaten, die während mehrerer Sensortrageperioden, also über mehrere Tage mit unterschiedlichen Sensorträgereinheiten 10, ermittelt wurden, zu speichern. Messdaten verschiedener Sensoren 3 können dabei jeweils mit einer Sensorkennung versehen werden, so dass Informationen wie die Messzeit für die einzelnen Messdatenwerte aus der Sensorkennung und der Tageszeit des Empfangs der Messdaten eines Sensors berechnet werden können.

Bei wiederholtem Zugriff des Anzeigegeräts 4 auf die Basisstation 2 werden bei dem dargestellten Ausführungsbeispiel nur zwischenzeitlich neu erzeugte Messdaten übertragen, um die Übertragungszeit und Sendeenergie zu minimieren. Der Zeitpunk des letzten Zugriffs kann in dem Anzeigegerät 4 in einem Datenheader gespeichert werden, in dem auch weitere allgemeine Daten, beispielsweise Kennungen der Sensorträgereinheit und Zeitformat, z.B Universal Time Convention, abgelegt werden können.

Der Speicher der Basisstation 2, in dem die verdichteten Messdaten gespeichert werden, kann mit einer Sperre versehen werden, die ein Überschreiben von Messdaten bei Anschluss einer neuen Sensorträgereinheit 10 erst zulässt, nachdem die Messdaten an das Anzeigegerät 4 gesendet und der Empfang der Daten bestätigt wurde. Beispielsweise kann die durch Anschließen einer neuen Sensorträgereinheit 10 bewirkte Inbetriebnahme der Basisstation 2 zunächst das Aussenden vorhandener Messdaten bewirken, bevor neue Messdaten aufgenommen werden. Möglich ist es aber auch, den Speicher der Basisstation 2 so groß zu wählen, dass mehr als die mit einer Sensorträgereinheit 10 in beispielsweise 5 Tagen, bevor ein Austausch erforderlich ist, erzeugten Messdaten in dem Speicher der Basisstation 2 gespeichert werden können. Eine automatische Übertragung von Messdaten vor Austausch einer Sensorträgereinheit 10 kann gewährleisten, dass für alle in der Basisstation 2 gespeicherten Messdaten von dem Anzeigegerät 4 eine Zeitmarke ermittelt werden kann. Werden nämlich nicht fortlaufend Messdaten erzeugt, besteht die Gefahr eines Datenverlustes hinsichtlich der Zuordnung zu einer eindeutigen Zeitmarke.

In Fig. 1 sind Anschlusskontakte 6a, 6b, 6c der Basisstation 2 zum Anschluss des Sensors 3 und Anschlusskontakte 7a, 7b der Basisstation 2 zum Anschluss der Batterie 5 dargestellt. Die Basisstation 1 hat ferner mindestens einen Dateneingang 8a, 8b zum Anschließen und Auslesen eines Datenträgers 11 mit Kalibrierungsdaten. Diese Kalibrierungsdaten kennzeichnen die Sensitivität des Sensors 3, bei dem es sich um einen elektrochemischen Sensor handelt, der beispielsweise ein Enzym enthält, das durch katalytische Umwandlung des Analyten Ladungsträger erzeugt, die durch einen Stromfluss zwischen den Elektroden des Sensors gemessen werden können. Handelt es sich bei dem Analyten beispielsweise um Glucose, kann das Enzym eine Glucoseoxidase sein.

Sensoren zur Messung von Analytkonzentrationen von Körperflüssigkeiten, beispielsweise Blut oder interstitielle Flüssigkeit, lassen sich nämlich in der Regel nicht mit exakt vorgegebenen Messsensitivitäten produzieren. Typischerweise treten zwischen Produktionschargen erhebliche Schwankungen der Sensorsensitivität auf, die bei der Auswertung von Messsignalen eines Sensors durch Kalibrierungsdaten berücksichtigt werden können. Die Kalibrierungsdaten werden üblicherweise vom Hersteller entweder an dem betreffenden Sensor selbst oder mittels Stichproben an anderen Sensoren der betreffenden Produktionscharge ermittelt. Derartige Kalibrierungsdaten beschreiben im Allgemeinen den Unterschied zwischen einer idealen Sensorsensitivität und einer ermittelten Sensorsensitivität.

Der Dateneingang 8a, 8b ist mit Federelementen 9 gekoppelt, die durch Federkraft das Anschließen eines Datenträgers erleichtern. Bevorzugt handelt es sich bei dem Datenträger 11 um einen Speicherchip, so dass der Dateneingang von elektrischen Anschlusskontakten gebildet wird. Der Datenträger 11 kann beispielsweise auch ein RFID oder ein magnetischer Datenträger sein und der Dateneingang 8a, 8b entsprechend einen Lesekopf umfassen. Die von dem Datenträger 11 gelesenen Kalibrierdaten werden bei dem beschriebenen Auswertungsbeispiel von der Basisstation 2 an das Anzeigegerät 4 gesendet und dort zur Auswertung der Messdaten verwendet. Möglich ist es auch, die Kalibrierdaten bereits in der Basisstation 2 zu verarbeiten und verdichtete Messdaten zu erzeugen, in denen die Kalibrierdaten berücksichtigt sind. Ein gefährliches Vertauschen von Datenträgern 11 mit Kalibrierdaten für unterschiedliche Sensoren 3 durch einen Benutzer kann so vermieden werden.

Die Basisstation 2 enthält eine Prüfschaltung 26, die an den Potentiostaten 48 angeschlossen ist und der Auswerteeinheit 47 der Basisstation 2 bei einem Systemtest ein Antwortsignal oder mehrere Antwortsignale liefert, die von der Auswerteeinheit 47 ausgewertet werden, wobei die Auswerteeinheit 47 einen Wert des mindestens einen Antwortsignals mit einem Erwartungswert vergleicht und ein Störungssignal erzeugt, wenn der Wert des mindestens einen Antwortsignals um mehr als einen vorgegebenen Toleranzwert von dem Erwartungswert abweicht. Dieses Störungssignal kann beispielsweise über den Sender 31 zu dem Anzeigegerät 4 gesendet werden, das einem Benutzer daraufhin die Störung anzeigt. Die Prüfschaltung simuliert einen an den Potentiostaten 48 angeschlossenen Sensor 3, so dass die Auswerteeinheit 47 anhand der Prüfschaltung 26 das Funktionieren des Potentiostaten 48 und den Ladezustand der Batterie 5 überprüfen kann. Im einfachsten Fall kann die Prüfschaltung 26 als ein umschaltbarer Festwiderstand ausgeführt sein.

Das Gehäuse 12 der Sensorträgereinheit 10 hat mindestens zwei getrennten Kammern 13, 14, 15, wobei in der ersten Kammer 13 unter sterilen Bedingungen der Sensor 3 und in einer zweiten Kammer 14 der Datenträger 11 mit Kalibrierungsdaten des Sensors 3 und gegebenenfalls eine Batterie 5 angeordnet sind. Bei dem dargestellten Ausführungsbeispiel ist in einer dritten Kammer 15 die Batterie 5 angeordnet.

Das Gehäuse 12 ist derart an eine Schnittstelle der Basisstation 2 angepasst, dass der in dem Gehäuse 12 enthaltene Sensor 3 und der dazugehörende Datenträger 11 an die Basisstation 2 durch Ansetzen des Gehäuses 12 an die Schnittstelle anschließbar sind. Durch Anschließen der Sensorträgereinheit 10 an die Basisstation 2 wird das Messsystem 1 automatisch initialisiert und der Messvorgang gestartet.

Das Gehäuse 12 enthält ein Federelement 20, dass beim Ansetzen des Gehäuses 12 an die Schnittstelle der Basisstation 2 das Anschließen der Batterie 5 unterstützt. In entsprechender Weise können auch in der ersten Kammer 13 und in der zweiten Kammer 14 Federelemente angeordnet sein, um das Anschließen des Sensors 3 und/oder des Datenträgers 11 an die Basisstation zu unterstützen.

Bei dem dargestellten Ausführungsbeispiel sind das Gehäuse 12 und die Schnittstelle der Basisstation 2 derart aufeinander abgestimmt, dass beim Ansetzen des Gehäuses 12 an die Schnittstelle zunächst die Batterie 5 und der Datenträger 11 an die Basisstation 2 angeschlossen werden und erst danach der Sensor 3 an die Basisstation 2 über die dafür vorgesehenen Kontakte 6a, 6b, 6c angeschlossen wird. Bei dem dargestellten Ausführungsbeispiel hat der Sensor 3 eine flache Struktur und wird mit einem Nullkraftstecker 3a an die Basisstation 2 angeschlossen. Der Sensor 3 kann beispielsweise auch eine Sandwichstruktur haben oder rotationssymmetrisch aufgebaut sein und die Kontakte 6a, 6b, 6c dazu passen ausgestaltet sein.

Eine Dichtung 21 der Basisstation 2, die bei dem darstellten Ausführungsbeispiel als Dichtring ausgeführt ist, sorgt dabei für eine wasserdichte Ankopplung des Sensors 3 an die Basisstation 2, so dass keine Feuchtigkeit in den Innenraum der Basisstation 2 eindringen kann. Die Basisstation 2 kann so beispielsweise auf dem Bauch eines Patienten getragen werden, ohne durch Körperflüssigkeiten beschädigt zu werden. Die Dichtung 21 bewirkt eine hochohmige Abdichtung der Basisstation 2 und des angeschlossenen Sensors 3. Auf diese Weise kann der Sensor 3 als elektrochemischer Sensor ohne Beeinträchtigung durch Leckströme mittels des in der Basisstation 2 angeordneten Potentiostaten 48 mit Spannung versorgt werden.

Fig. 3 zeigt in einer Schrägansicht ein Ausführungsbeispiel der Basisstation 2 mit daran angeschlossener Sensorträgereinheit 10. Fig. 4 zeigt eine Querschnittsansicht zu Fig. 3, in der die sterile Gehäusekammer 13 mit dem darin angeordneten Sensor 3 sowie die zweite Gehäusekammer 14 mit darin angeordneter Batterie 5 und Datenträger 11 der Sensorträgereinheit 10 dargestellt sind. Zu sehen ist ferner, dass die Basisstation 2 einen Potentiostaten 48 zur Strom- und Spannungsversorgung des Sensors 3 und eine als Mikroprozessor ausgebildete Auswerteeinheit 47 sowie einen Sender und Empfänger 31 zur drahtlosen Kommunikation mit dem Anzeigegerät 4 enthält, wobei die Antenne oder Antennen zur drahtlosen Kommunikation mit dem Anzeigegerät 4 von dem Gehäuse der Basisstation 2 umbeben sind.

Das Gehäuse 12 der Sensorträgereinheit 10 ist ebenso wie das Gehäuse der Basisstation 2 aus Hartplastik gefertigt. Die Schnittstellen der Basisstation 2 und der Sensorträgereinheit 10 sind bei dem dargestellten Ausführungsbeispiel für eine formschlüssige Verbindung ausgelegt. Das Gehäuse 12 weist Rastzapfen 40 auf, die in dazu passende Ausnehmungen der Basisstation 2 eingreifen. Diese Ausnehmungen sind an den Außenseiten von zwei Federschenkeln 41 angeordnet, so dass die Rastzapfen durch Federkraft in die Ausnehmungen gedrückt werden. Die Federschenkel 41 können zusammengedrückt werden, so dass sich die Rastzapfen 40 der Sensorträgereinheit 10 aus den dazu passenden Ausnehmungen lösen und die Sensorträgereinheit 12 von der Basisstation 2 abgenommen werden kann. In entsprechender Weise kann bei zusammengepressten Federschenkeln 41 die Sensorträgereinheit 12 an die Basisstation 2 angesetzt werden.

Alternativ oder zusätzlich zu einer formschlüssigen Verbindung kann die Sensorträgereinheit 10 auch so ausgebildet werden, dass sie klemmschlüssig mit der Basisstation 2 verbindbar ist.

Die in Fig. 4 dargestellte Querschnittsansicht zeigt, dass die Sensorträgereinheit 10 zwei getrennte Kammern 13, 14 aufweist, wobei in einer ersten Kammer 13 unter sterilen Bedingungen der Sensor 3 und in der zweiten Kammer 14 ein Datenträger 11 mit Kalibrierungsdaten des Sensors 3 und eine Batterie 5 zur Stromversorgung der Basisstation 2 angeordnet sind. Anschlussleitungen des Sensors 3 führen aus der ersten Kammer 13 in die zweite Kammer 14 zu einer Leiterplatte 45, die mit dem als Speicherchip ausgeführten Datenträger 11 kontaktiert ist. Die Leiterplatte 45 ist über eine Steckverbindung 46, im dargestellten Ausführungsbeispiel eine mehrpolige Steckverbindung, an die Basisstation 2 angeschlossen.

Die sterile Kammer 13, in welcher der Sensor 3 gelagert ist, ist von zwei Septen 42 verschlossen, wobei eine Insertionsnadel 43 zum Einführen des Sensors 3 in einen menschlichen oder tierischen Körper durch die Septen 42 hindurchgeführt ist. Das aus der Gehäusekammer 13 herausragende Vorderende der Insertionsnadel 43 ist von einer Sterilschutzkappe 44 umgeben, die erst abgenommen wird, wenn der Sensor 3 mittels der Insertionsnadel 43 in einen menschlichen oder tierischen Körper eingeführt werden soll. Die Sterilschutzkappe 44 ist bei dem dargestellten Ausführungsbeispiel über eine Sollbruchstelle 16 mit dem übrigen Gehäuse 12 verbunden.

Für den Tragekomfort ist es günstig, wenn die Sensorträgereinheit 10 eine Sensoröffnung, durch die der Sensor 3 bzw. dessen Anschlussleitung 39 geführt ist, aufweist, die möglichst weit von dem Rand des Bodens 27 der Sensorträgereinheit 10 entfernt ist, um Kippmomente zu minimieren. Bei dem dargestellten Ausführungsbeispiel ist die Sensoröffnung mehr als 1 cm vom Rand des Bodens 27 entfernt angeordnet. Möglich ist es insbesondere auch, die Sensoröffnung in der Mitte der Grundplatte 27 anzuordnen, bzw. in einer Position, die weniger als 20 % der Länge der Grundplatte von der Mitte abweicht, insbesondere weniger als 10 % der Länge der Grundplatte von der Mitte abweicht.

Zur Insertion des Sensors 3 wird die Sensorträgereinheit 10 beispielsweise auf dem Bauch eines Patienten angeordnet und die Insertionsnadel 43 in den Körper des Patienten eingestochen. Anschließend kann die Insertionsnadel 43, die beispielsweise als eine den Sensor 3 tragende Rinne ausgeführt sein kann, wieder aus dem Körper des Patienten herausgezogen, wobei der Sensor 3 im Körper des Patienten verbleibt. Die Insertion kann prinzipiell unter einem beliebigen Winkel der Einstichrichtung zur Hautoberfläche erfolgen. Besonders vorteilhaft für eine Insertion in Unterhautfettgewebe sind Winkel zwischen 30° und 60°. Der Sensor 3 hat einen zur Implantation bestimmten Sensorkopf 38 und eine an den Sensorkopf 38 angeschlossenen elektrische Anschlussleitung 39, wobei die Anschlussleitung in dem Gehäuse 12 der Sensorträgereinheit 10 gebogen geführt ist, vorzugsweise um 30° bis 150° gebogen ist. Die dargestellte Sensorträgereinheit 10 ist derart ausgebildet, dass ein Benutzer bei der Insertion eines Sensors 3 die Einstichstelle sehen kann.

Die Sensorträgereinheit 10 hat eine Grundplatte 27, die bestimmungsgemäß auf den Körper des Patienten geklebt wird. Die Basiseinheit 2 wird auf die Sensorträgereinheit 10, genauer gesagt auf die Grundplatte 27, aufgesetzt. Das Gehäuse der Basisstation 2 liegt dann seitlich an dem Gehäuse 12 der Sensorträgereinheit 10 an. Die Grundplatte 27 weist eine Klebefläche auf, die durch eine abziehbare Folie geschützt ist, die mehrteilig ist und deren Teile jeweils unterschiedliche Bereiche der Klebefläche bedecken und beim Abziehen voneinander getrennt werden. Diese Klebefläche kann beispielsweise als eine beidseitig klebende Folie oder ein beidseitig klebendes Kissen ausgebildet sein, so dass eine Klebefläche mit der Unterseite der Sensorträgereinheit 10 und die andere Klebefläche bestimmungsgemäß mit der Haut eines Patienten verklebt werden kann. Die Folien zum Schutz der Klebeflächen können wie bei handelsüblichen Pflastern ausgebildet sein. Indem die Klebeflächen durch mehrteilige Folien geschützt werden, können die Folien leichter von den Klebeflächen entfernt werden. Auf diese Weise ist es beispielsweise möglich, das Festkleben der Sensorträgereinheit 10 erst nach dem Insertieren des Sensors vorzunehmen, da die einzelnen Teile der die Klebeflächen schützenden Folien aus unterschiedlichen Richtungen unter der Sensorträgereinheit 10 herausgezogen werden können.

Bevorzugt ist der Träger der Klebefläche, beispielsweise eine Folie oder ein Kissen, dehnbar, so dass der Träger später vom Körper des Patienten auch durch Zug, dessen Kraftvektor parallel zur Ebene der Klebefläche verläuft, entfernt werden kann. Klebekissen mit entsprechenden elastischen Eigenschaften sind im Handel beispielsweise zum Befestigen Postern unter der Marke tesa powerstrips erhältlich.

Ein zum Befestigen der Sensorträgereinheit 10 geeigneter Träger der Klebefläche kann beispielsweise aus einem Schaumstoff, insbesondere einem Polyurethanschaum, hergestellt werden. Ein Träger aus Schaumstoff hat zudem den Vorteil, Relativbewegungen des Körpers relativ zu der Sensorträgereinheit 10 zu reduzieren.

Bei der Herstellung der Sensorträgereinheit 10 wird der Sensor 3 zunächst in der ersten Gehäusekammer 13 angeordnet und diese versiegelt. Zur Herstellung des in Fig. 4 dargestellten Ausführungsbeispiels wird bei diesem Arbeitsschritt auch eine Sterilschutzkappe 44 um das aus der ersten Gehäusekammer 13 herausragende Ende des Sensors 3 sowie die den Sensor 3 tragende Insertionsnadel 43 angeordnet und die Sterilschutzkappe 44 mit der Gehäusekammer 13 verbunden. Anschließend wird die Gehäusekammer 13 intensiver Strahlung, beispielsweise Elektronenstrahlung, ausgesetzt, so dass der Sensor 3 und die Insertionsnadel 43 sterilisiert werden. In Fig. 4 ist eine Detailansicht der ersten Gehäusekammer 13 mit der daran angebrachten Sterilschutzkappe 44 dargestellt, die nach Einbringen des Sensors 3 zusammen durch Bestrahlen sterilisiert werden.

In einem weiteren Arbeitsschritt wird die erste Gehäusekammer 13 mit der zweiten Gehäusekammer 14 zusammengefügt, um die Sensorträgereinheit 10 zu schaffen. Dabei kann an der Sensorträgereinheit 10 eine Insertionshilfe 37 angebracht werden, die das Einstechen des Sensors 3 bzw. einer ihn tragenden Insertionskanüle 43 in den Körper des Patienten erleichtert. Die Insertionshilfe kann eine Feder umfassen, die bei Auslieferung der Sensorträgereinheit bereits vorgespannt sein kann oder mit einem Vorspannhebel (nicht dargestellt) vom Benutzer gespannt werden kann. Durch Betätigen eines Auslöseknopfes kann ein Benutzer die gespannte Insertionsfeder auslösen und einen Einstich des Sensors bzw. einer ihn tragenden Insertionskanüle 43 bewirken.

### Bezugszeichenliste

- 1: System zur in-vivo Messung einer Analytkonzentration
- 2: Basisstation
- 3: Sensor
- 3a, 3b, 3c: Sensorkontakte
- 4: Anzeigegerät
- 5: Batterie
- 6a, 6b, 6c: Anschlusskontakte
- 7a, 7b: Anschlusskontakte
- 8a, 8b: Dateneingang der Basisstation
- 9: Federelement
- 10: Sensorträgereinheit
- 11: Datenträger
- 12: Gehäuse
- 13: Gehäusekammer
- 14: Gehäusekammer
- 15: Gehäusekammer
- 16: Sollbruchstelle
- 17: Gehäuseteil
- 20: Federelement
- 21: Dichtung
- 26: Prüfschaltung
- 27: Grundplatte
- 28: Teststreifen
- 29: Anzeigeeinrichtung
- 30: Sender/Empfänger
- 31: Sender/Empfänger
- 32: Messsignale
- 33: Analog-Digital Konverter
- 34: Umschalter
- 35: Speicher
- 36: verdichtete Messdaten
- 37: Insertionshilfe
- 38: Sensorkopf
- 39: Anschlussleitung
- 40: Rastzapfen
- 41: Federschenkel der Basisstation 2
- 42: Septa
- 43: Insertionsnadel
- 44: Sterilschutzkappe
- 45: Leiterplatte
- 46: Steckverbindung
- 47: Auswerteeinheit
- 48: Potentiostat
- 49: Spannung
- 50: Kalibrierungsdaten
- 51: Rohdaten
- 52: Auswerteeinheit
- 53: Analytkonzentrationswerte
- 54: Speicher
- 55: Echtzeituhr
- 56: Messgerät
- 60: Zwischenspeicher
- 61: Sender

## Patentansprüche

1. System zur in-vivo Messung einer Analytkonzentration in einem menschlichen oder tierischen Körper, mit
mindestens einem implantierbaren Sensor (3) zum Erzeugen von mit der zu messenden Analytkonzentration korrelierenden Messsignalen,
einer an den Sensor (3) anschließbare Basisstation (2), die eine elektronische Auswerteeinheit (47) zum Auswerten von Messsignalen eines angeschlossenen Sensors (3) und einen Sender (31) zum drahtlosen Übertragen von Auswertungsergebnissen enthält, und
einem Anzeigegerät (4), das einen Empfänger zum Empfangen der von der Basisstation (2) gesendeten Auswertungsergebnisse und eine Anzeigeeinrichtung (29) zum Anzeigen von Analytkonzentrationswerten aufweist,
**dadurch gekennzeichnet, dass**
dass der Sensor (3) Teil einer austauschbaren Sensorträgereinheit (10) ist, die ein Gehäuse (12) mit einer Schnittstelle zum Anschließen des Sensors (3) an die Basisstation (2) aufweist, wobei die Basisstation (2) ein an die Sensorträgereinheit (10) angepasstes Gehäuse aufweist, das eine Schnittstelle aufweist, die zu der Schnittstelle der Sensorträgereinheit (10) passt, so dass der Sensor (3) durch Ansetzten des Gehäuses der Basisstation (2) an das Gehäuse (12) der Sensorträgereinheit (10) an die Auswerteeinheit (47) der Basisstation (2) angeschlossen wird.

2. System nach einem der vorhergehenden Ansprüche, **dadurch gekennzeichnet, dass** die Basisstation (2) mit der Sensorträgereinheit (10) verrastet.

3. System nach einem der vorhergehenden Ansprüche, **dadurch gekennzeichnet** das die Sensorträgereinheit (10) eine Batterie (5) zur Stromversorgung der Basisstation (2) enthält.

4. System nach einem der vorhergehenden Ansprüche, **dadurch gekennzeichnet, dass** das Gehäuse der Sensorträgereinheit (10) eine Sollbruchstelle (16) zum Entfernen eines Gehäuseteils (44), der eine den Sensor (3) enthaltende Kammer (13) verschließt, aufweist.

5. System nach einem der vorhergehenden Ansprüche, **dadurch gekennzeichnet, dass** die Sensorträgereinheit (10) bestimmungsgemäß auf den Körper eines Patienten geklebt und die Basiseinheit (2) auf Sensorträgereinheit (10) aufgesetzt wird.

6. System nach einem der vorhergehenden Ansprüche, **dadurch gekennzeichnet, dass** die Auswerteeinheit (47) der Basisstation (2) im Betrieb von einem angeschlossenen Sensor (3) gelieferte Rohdaten (33) statistisch auswertet und aus den Rohdaten (33) verdichtete Messdaten (36) erzeugt, die über den Sender (31) zu dem Anzeigegerät (4) übertragen werden, und das Anzeigegerät (4) eine elektronische Auswerteeinheit (52) enthält, die im Betrieb durch Auswertung der verdichteten Messdaten (36) mindestens einen Analytkonzentrationswert ermittelt.

7. System nach Anspruch 6, **dadurch gekennzeichnet, dass** die elektronische Auswerteeinheit (52) des Anzeigegeräts (4) bei der Auswertung der verdichteten Messdaten (36) eine Kalibrierung durchführt.

8. System nach Anspruch 6 oder 7, **dadurch gekennzeichnet, dass** die verdichteten Messdaten (36) fortlaufend für konstante Zeitintervalle ermittelt werden und in dem Anzeigegerät (4) aus einem Übertragungszeitpunkt, der Länge der Zeitintervalle und deren Reihenfolge für die verdichteten Messdaten (36) Zeitmarken berechnet werden, die Datum und Uhrzeit enthalten.

9. System nach einem der vorhergehenden Ansprüche, **dadurch gekennzeichnet, dass** das Anzeigegerät (4) eine Messeinrichtung zur Bestimmung der Analytkonzentration einer Körperflüssigkeitsprobe enthält.

10. System nach Anspruch 9, **dadurch gekennzeichnet, dass** die Auswerteeinheit (52) des Anzeigegeräts (4) an die Messeinrichtung angeschlossen ist und die mittels der Messeinrichtung an Körperflüssigkeitsproben bestimmten Analytkonzentrationswerte bei der Auswertung der von der Basisstation (2) übermittelten verdichteten Messdaten (36) zur Kalibrierung verwendet.

11. System nach einem der vorhergehenden Ansprüche, **dadurch gekennzeichnet, dass** die Auswerteeinheit (47) der Basisstation (2) bei der statistischen Auswertung zum Erzeugen der verdichteten Messdaten (36) ein Verfahren ausführt, bei dem aus den in einem Zeitintervall erzeugten Messsignalen (32) eines Sensors (3) Paare von Messsignalwerten gebildet werden, danach für jedes Messsignalwertepaar eine Steigung für eine die die beiden Werte des Wertepaares verbindende Linie ermittelt wird, danach ein Medianwert der ermittelten Steigungen berechnet wird und danach für das Zeitintervall ein verdichteter Messdatenwert aus dem Medianwert der Steigung und dem verdichteten Messdatenwert des vorhergehenden Zeitintervalls berechnet wird.

12. System nach einem der vorhergehenden Ansprüche, **dadurch gekennzeichnet, dass** die Basisstation (2) einen Potentiostaten (48) zur Spannungsversorgung eines Sensors (3) einer angeschlossenen Sensorträgereinheit (10) enthält.

13. System nach einem der vorhergehenden Ansprüche, **dadurch gekennzeichnet, dass** die Basisstation (2) eine Prüfschaltung (26) enthält, die an den Potentiostaten (48) angeschlossen ist und der Auswerteeinheit (48) der Basisstation (2) bei einem Systemtest ein Antwortsignal liefert, das von der Auswerteeinheit (48) ausgewertet wird, wobei die Auswerteeinheit (48) einen Wert des Antwortsignals mit einem Erwartungswert vergleicht und ein Störungssignal erzeugt, wenn der Wert des Antwortsignals um mehr als einen vorgegebenen Toleranzwert von dem Erwartungswert abweicht.

14. System nach einem der vorhergehenden Ansprüche, **dadurch gekennzeichnet, dass** die Sensorträgereinheit (10) eine Grundplatte (27) aufweist, die bestimmungsgemäß auf den Körper eines Patienten geklebt wird und eine angeschlossene Basisstation (2) trägt, wobei das Gehäuse der Basisstation (2) seitlich an dem Gehäuse (12) der Sensorträgereinheit (10) anliegt.

15. System nach einem der vorhergehenden Ansprüche, **dadurch gekennzeichnet, dass** die Sensorträgereinheit (10) eine Klebefläche aufweist, die durch eine abziehbare Folie geschützt ist, die mehrteilig ist und deren Teile jeweils unterschiedliche Bereiche der Klebefläche bedecken und beim Abziehen voneinander getrennt werden.

16. System zur in-vivo Messung einer Analytkonzentration in einem menschlichen oder tierischen Körper, mit
mindestens einem implantierbaren Sensor (3) zum Erzeugen von mit der zu messenden Analytkonzentration korrelierenden Messsignalen,
einer an den Sensor (3) anschließbare Basisstation (2), die eine elektronische Auswerteeinheit (47) zum Auswerten von Messsignalen eines angeschlossenen Sensors (3) und einen Sender (31) zum drahtlosen Übertragen von Auswertungsergebnissen enthält, und
einem Anzeigegerät (4), das einen Empfänger zum Empfangen der von der Basisstation (2) gesendeten Auswertungsergebnisse und eine Anzeigeeinrichtung (29) zum Anzeigen von Analytkonzentrationswerten aufweist, **dadurch gekennzeichnet, dass** die Auswerteeinheit (47) der Basisstation (2) im Betrieb die von einem angeschlossenen Sensor (3) gelieferten Messsignale als Rohdaten statistisch auswertet und aus den Rohdaten verdichtete Messdaten erzeugt, die über den Sender (31) zu dem Anzeigegerät (4) übertragen werden, und das Anzeigegerät (4) eine elektronische Auswerteeinheit enthält, die im Betrieb durch Auswertung der verdichteten Messdaten einen Analytkonzentrationswert ermittelt.

17. System zur in-vivo Messung einer Analytkonzentration in einem menschlichen oder tierischen Körper, mit
mindestens einem implantierbaren Sensor (3) zum Erzeugen von mit der zu messenden Analytkonzentration korrelierenden Messsignalen,
einer an den Sensor (3) anschließbare Basisstation (2), die eine elektronische Auswerteeinheit (47) zum Auswerten von Messsignalen eines angeschlossenen Sensors (3) und einen Sender (31) zum drahtlosen Übertragen von Auswertungsergebnissen enthält, und
einem Anzeigegerät (4), das einen Empfänger zum Empfangen der von der Basisstation (2) gesendeten Auswertungsergebnisse und eine Anzeigeeinrichtung (29) zum Anzeigen von Analytkonzentrationswerten aufweist, **dadurch gekennzeichnet, dass** der Sensor Teil einer Sensorträgereinheit (10) ist, die eine Grundplatte (27) aufweist, die bestimmungsgemäß auf den Körper eines Patienten geklebt wird und eine angeschlossene Basisstation (2) trägt, wobei das Gehäuse der Basisstation (2) an dem Gehäuse (12) der Sensorträgereinheit (10) anliegt.
